# EUROPEAN PATENT APPLICATION

(11) **EP 3 944 853 A1**
(43) Date of publication of application: **02.02.2022**
(21) Application number: 20188906.0
(22) Date of filing: 31.07.2020
(51) Int. Cl.: A61K 8/34, A61K 8/36, A61K 8/365, A61K 8/368, A61Q 11/00

(54) **LIQUID L-MENTHOL COMPOSITION**

(71) Applicant: BASF SE, 67056 Ludwigshafen am Rhein (DE)
(72) Inventor: KOLTER, Karl., 67117 Limburgerhof (DE); GUTH, Felicitas., 67056 Ludwigshafen (DE); KRAUSE, Wolfgang., 68623 Lampertheim (DE); PELZER, Ralf., 68623 Lampertheim (DE)
(74) Representative: BASF IP Association

(57) **Abstract**

The presently claimed invention relates to a composition of L-menthol and at least one hydrogen bond donor wherein the composition is liquid at normal temperature and pressure. The presently claimed invention further relates to a process for preparing such compositions and products comprising the aforesaid L-menthol composition with further ingredients.

## Description

### Filed of invention

The presently claimed invention relates to a composition of L-menthol and at least one hydrogen bond donor, wherein the composition is liquid at normal temperature and pressure. The presently claimed invention further relates to a process for preparing such compositions and products comprising the aforesaid L-menthol composition with further ingredients.

### Background of the invention

Menthol is a naturally occurring active ingredient that is widely used in the pharmaceutical, cosmetics and food industries. In natural sources, for example the peppermint oil, menthol occurs in the form of four diastereomeric enantiomeric pairs, of which only the main component, the (-)-menthol or L-menthol, has the desired flavour and other sensory properties.

For a long time, it has been known that L-menthol can solidify in four different crystalline modifications which, for the same chemical composition, have different physical properties, as already described in J. Am. Chem. Soc., Vol. 39 (8), 1917, pp. 1515 to 1525. Thus, in particular the melting points of these different modifications lie between 33 °C and 43 °C, as described in Ar-chiv der Pharmazie, 307 (7), 1974, pp. 497 to 503. The melting point of the stable alpha-modification is accordingly 42 to 43 °C.

On account of these melting points, L-menthol can be supplied to the end consumer either as a melt kept liquid in heated containers, or else in the form of crystals or other solidified moldings. Generally, all solids which, like L-menthol, have a melting point that is only just above the ambient temperature, have a high tendency to cake and to agglomerate. However, the processing of such caked material is associated with considerable undesired additional expenditure. Should then pure L-menthol, i.e. menthol which not treated with auxiliaries, such as, for example release agents, be sold as solid, it must be ensured, either by virtue of a closed cooling chain or by virtue of the type of molding, that the product reaches the end consumer in a pourable form.

Commercially, menthol is available in the form of large crystals which, for a length of 0.5 to 3 cm, have a thickness of from 1 to 3 mm. They are traditionally cultivated in small amounts from naturally obtained peppermint oil by crystallizing the oil in troughs or vats over many days in cooling houses. These crystals have good pourability only in the case of a small bed height, but cake appreciably under increased load and/or at elevated temperature. The technical expenditure of the crystallization, separation and purification of the crystals and the low space-time yield of such a lengthy process make it unattractive for industrial application.

L-menthol is commercially supplied to the intermediate or final consumer in liquid form by maintaining the container at a specific temperature or in the form of crystal needles.

Based on the foregoing, there still exists a need to provide L-menthol in liquid form which offers reduced manufacturing costs, reduced shipping costs and better handling properties as compared to the flaked or the crystalline material.

Thus, the object of the presently claimed invention is to provide menthol in liquid form which can be easily metered, directly used for liquid fragrances or aroma mixtures, and can be easily transported without the need to use special transportation means or equipment.

### Summary of the invention

Surprisingly, it was found that certain compounds significantly decrease the melting point of menthol and are capable of forming eutectic mixtures, whereby L-menthol can be maintained in liquid state for a long period.

Thus, in one aspect, the presently claimed invention relates to a composition comprising:
a) L-menthol,
b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
   wherein the molar ratio of a) to b) in the range of 10:1 to 1:1

In a further aspect the presently claimed invention relates to a composition wherein the composition further comprises water in the range of ≥ 0.01wt.% to ≤ 5 wt. % to the total weight of the composition.

In another aspect, the presently claimed invention relates to a composition comprising:
a) L-menthol,
b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
   wherein the molar ratio of a) to b) in the range of 10:1 to 1:1 and,
c) water in the range of ≥ 0.01wt.% to ≤ 5 wt. % to the total weight of the composition,
   wherein the composition remains in a liquid state in a temperature range of 22°C to 50° C at normal atmospheric pressure.

In a further aspect, the presently claimed invention relates to a method for maintaining L-menthol in a liquid state comprising at least the step of:
(I) mixing L-menthol with at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa ½, wherein the molar ratio of a) to b) in the range of 10:1 to 1:1 and optionally with water that the obtained mixture has a water content in the range of ≥ 0.01wt.% to ≤ 5 wt. % of the total weight of the mixture

In another aspect, the presently claimed invention relates to a method for maintaining L-menthol in a liquid state at a temperature range of 22°C to 50° C at normal atmospheric pressure for a period of more than 1 year which comprises at least the step of:
(I) mixing L-menthol with at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa ½,
   in a molar ratio of 10:1 to 1:1.

In yet another aspect, the presently claimed invention relates to a process for preparing the composition as describe above.

In a further aspect, the presently claimed invention relates to use of the composition in flavoring material preparations, cosmetic preparations, preparations used for nutrition, oral hygiene preparations, dental hygiene preparations, perfumes, pharmaceutical preparation, dermatological product preparation, encapsulated menthol-containing preparations, and household product preparations.

In another aspect, the presently claimed invention relates to a product containing the composition according to the presently claimed invention, wherein the product is selected from the group consisting of flavoring products, cosmetic products, nutritional products, oral hygiene products, dental hygiene products, perfumes, pharmaceutical products, dermatological products, encapsulated menthol-containing products, and household products.

### Detailed description of the invention

Before the present compositions and formulations of the presently claimed invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well i.e. a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment but may.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

### Composition

In an embodiment, the presently claimed invention relates to a composition comprising:
a) L-menthol,
b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
   wherein the molar ratio of a) to b) in the range of 10:1 to 1:1

In a further embodiment, the presently claimed invention relates to a composition wherein the composition further comprises water in the range of ≥ 0.01wt.% to ≤ 5 wt. % to the total weight of the composition.

In an embodiment, the presently claimed invention relates to a composition comprising:
a) L-menthol,
b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
   wherein the molar ratio of a) to b) in the range of 10:1 to 1:1 and,
c) water in the range of ≥ 0.01wt.% to ≤ 5 wt. % to the total weight of the composition.

In another aspect, the presently claimed invention relates to a composition comprising:
a) L-menthol,
b) at least one hydrogen bond donor having Hansen solubility parameters in the range of δH ≥ 8.0 to ≤ 30.0 MPa½, δD ≥ 16.0 to ≤ 21.0 MPa½ and δP ≥ 2.5 to ≤ 14.0 MPa½,
   wherein the molar ratio of a) to b) in the range of 10:1 to 1:1 and,
c) water in the range of ≥ 0.01wt.% to ≤ 5 wt. % to the total weight of the composition,
   wherein the composition remains in a liquid state in a temperature range of 22°C to 50° C at normal atmospheric pressure.

In yet another embodiment, the presently claimed invention relates to a composition comprising:
a) L-menthol,
b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
   wherein the molar ratio of a) to b) in the range of 10:1 to 1:1,
   wherein L-menthol and the at least one hydrogen bond donor form a eutectic mixture

In yet another embodiment, the presently claimed invention relates to a composition comprising:
a) L-menthol,
b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
   wherein the molar ratio of a) to b) in the range of 10:1 to 1:1 and,
c) water in the range of ≥ 0.01wt.% to ≤ 5 wt. % to the total weight of the composition,
   wherein L-menthol and the at least one hydrogen bond donor form a eutectic mixture.

### L-menthol

Suitable starting materials used for the composition are melts of L-menthol of the formula (I), wherein the molten menthol may be of natural or synthetic origin and has an enantiomeric excess of usually at least 95, 96 or 97% ee to 100% ee, preferably 98, 98.5 or 99 to 99.9% ee. Particularly suitable starting materials in the process according to the invention are those melts of L-menthol which have a content of L-menthol of at least 95, 96 or 97% by weight or more, preferably at least 98 to 100% by weight and very particularly preferably 98, 98.5 or 99 to 99.9 wt.% (in each case based on the total weight of the melt), in addition to impurities such as residues of solvents, diastereomers of L-menthol of the formula (I) or by-products of synthesis or isolation.

### Hansen parameter

The ability of a solvent to dissolve a given substance, e.g. a L-menthol, may conveniently be evaluated by parameter consideration according to the "Hansen system", which is described in "Hansen Solubility Parameters -A Users Handbook", published by CRC Press (2000). According to the Hansen system, a solvent or mixture of solvents may be described by three solubility parameters 5D (dispersion parameter), δP (polarity parameter) and δH (hydrogen bonding parameter). Different solvents with regard to Hansen solubility parameters and molecular structures have been found to be particular useful as solvents.

### Hydrogen bond donor

A hydrogen bond donor is a compound which acts as a donor in a hydrogen bond, i.e. the hydrogen bond donor contains a donor atom to which the hydrogen atom participating in the hydrogen bond is covalently bonded. This donor atom is usually a strongly electronegative atom.

According to the invention the at least one hydrogen bond donor has Hansen solubility parameters of δH ≥ 8.0 MPa ½, δD ≥ 16.0 MPa½ and δP ≥ 2.5 MPa½.

Preferably, the at least one hydrogen bond donor has Hansen solubility parameters in the range of 5H ≥ 8.0 to ≤ 30.0 MPa ½,
δD≥ 16.0 to ≤ 21.0 MPa½ and δP ≥ 2.5 to ≤ 14.0 MPa½.

More preferably, the at least one hydrogen bond donor has Hansen solubility parameters in the range of δH ≥ 8.0 to ≤ 30.0 MPa ½,
δD ≥ 16.0 to ≤ 18.0 MPa½ and δP ≥ 2.5 to ≤ 12.0 MPa½.

In a particularly preferred embodiment of the presently claimed invention, the at least one hydrogen bond donor has Hansen solubility parameters δH 28.4 MPa ½, 5D 17.0 MPa½ and δP 8.3MPa½.

In another particularly preferred embodiment of the presently claimed invention, the at least one hydrogen bond donor has Hansen solubility parameters δH 8.7 MPa ½, 5D 16.2 MPa½ and δP ≥ 11.2MPa½.

Preferably, the at least one hydrogen bond donor is selected from the group consisting of C2-C3 aliphatic acids, aromatic carboxylic acids, phenols and C3-C4 aliphatic diols.

The C2-C3 aliphatic acids as hydrogen bond donors are preferably selected from the group consisting of acetic acid, oxalic acid, glycolic acid, propionic acid, malonic acid, pyruvic acid, D-L-lactic acid and L-lactic acid.

More preferably, the C2-C3 aliphatic acids as hydrogen bond donor are selected from the group consisting of pyruvic acid, D-L-lactic acid and L-lactic acid.

In a particularly preferred embodiments of the presently claimed invention, the C2-C3 aliphatic acid as hydrogen bond donors is pyruvic acid.

In another particularly preferred embodiment of the presently claimed invention, the C2-C3 aliphatic acid as hydrogen bond donor is D-L-lactic acid
In another particularly preferred embodiment of the presently claimed invention, the C2-C3 aliphatic acid as hydrogen bond donor is L-lactic acid
The aromatic carboxylic acids as hydrogen bond donors are preferably selected from the group consisting of benzoic acid, toluic acid, phthalic acid and salicylic acid.

More preferably, the aromatic carboxylic acid as hydrogen bond donor is benzoic acid.

The phenols as hydrogen bond donors are preferably selected from the group consisting of propyl gallate, butylated hydroxy anisole and butylated hydroxy toluene.

More preferably, the phenol as hydrogen bond donor is butylated hydroxy anisole.

The C3-C4 aliphatic diols as hydrogen bond donors are preferably selected from the group consisting of 1,2-butanediol, 1,3-butanediol, 1,4-butanediol, 2,3-butandiol, 1,3-propanediol and 1,2-propanediol.

More preferably, the C3-C4 aliphatic diol as hydrogen bond donor is 1,2-propanediol.

Preferably, the amount of the at least one hydrogen bond donor is in the range of ≥ 4.5 wt.% to ≤ 25 wt.%, based on the total weight of the composition.

More preferably, the amount of the at least one hydrogen bond donor is in the range of ≥ 9.5 wt. % to ≤ 20 wt. %, based on the total weight of the composition.

Therefore, the amount of the at least one hydrogen bond donor is preferably 10, 11, 12, 13, 14, 15, 16, 17, 18, 19 or 20 wt. %, based on the total weight of the composition.

### Molar ratio

The molar ratio of the L-menthol to the at least one hydrogen bond donor is in the range of 10:1 to 1:1.

Preferably, the molar ratio of the L-menthol to the at least one hydrogen bond donor is in the range of 8:1 to 2:1.

More preferably, the molar ratio of the L-menthol to the at least one hydrogen bond donor is in the range of 6:1 to 3:1.

In a particularly preferred embodiment of the presently claimed invention, the molar ratio of the L-menthol to lactic acid is in the range of 6:1 to 3:1.

In another particularly preferred embodiment of the presently claimed invention, the molar ratio of the L-menthol to pyruvic acid, is in the range of 6:1 to 3:1.

### Water content

Preferably the water content in the composition is in the range of ≥ 0.01wt.% to ≤ 5 wt. % to the total weight of the composition.

More preferably, the water content in the composition is in the range of ≥ 0.01wt.% to ≤ 4 wt. % to the total weight of the composition, more preferably in the range of ≥ 0.01wt.% to ≤ 3 wt. % to the total weight of the composition, even more preferably ≥ 0.01wt.% to ≤ 2.5 wt. %, to the total weight of the composition.

### Amount of L- menthol

Preferably, the amount of L-menthol is in the range of ≥ 70 wt. % to ≤ 95 wt. %, based on the total weight of the composition.

More preferably, the amount of L-menthol is in the range of ≥ 75 wt. % to ≤ 90 wt. %, based on the total weight of the composition.

Therefore, the amount of L-menthol is preferably 75, 76, 77, 78, 79, 80, 81, 82, 83, 84, 85, 86, 87, 88, 89 or 90 wt. %, based on the total weight of the composition.

### Temperature:

Preferably, the composition remains in a liquid state at a temperature in the range from 22°C to 80°C at normal atmospheric pressure. More preferably the composition remains in a liquid sate at a temperature in the range from 22°C to 70°C at normal atmospheric pressure. Most preferably, the composition remains in a liquid state at a temperature in the range from 22°C to 50°C at normal atmospheric pressure.

### Stability:

In another embodiment of the presently claimed invention, the composition remains in a liquid state at a temperature in the range of 22°C to°50 C at normal atmospheric pressure for a period of at least 1 year.

In another embodiment of the presently claimed invention, the composition remains in a liquid state at a temperature in the range of 22°C to°50 C at normal atmospheric pressure for a period of at least 6 months.

In another embodiment of the presently claimed invention, the composition remains in a liquid state at a temperature in the range of 22°C to°50 C at normal atmospheric pressure for a period of at least 3 months.

In a preferred embodiment, the presently claimed invention relates to a composition comprising:
a) L-menthol, and
b) lactic acid,
   wherein the molar ratio of a) to b) in the range of 6:1 to 3:1, and optionally,
c) water in the range of ≥ 0.01wt.% to ≤ 5 wt. % to the total weight of the composition,

In another preferred embodiment, the presently claimed invention relates to a composition comprising:
a) L-menthol, and
b) lactic acid,
   wherein the molar ratio of a) to b) in the range of 6:1 to 3:1, and optionally
c) water in the range of ≥ 0.01wt.% to ≤ 5 wt. % to the total weight of the composition,
   wherein the composition remains in a liquid state at a temperature in the range of 22°C to°50 C at normal atmospheric pressure.

In another preferred embodiment, the presently claimed invention relates to a composition comprising:
a) L-menthol, and
b) pyruvic acid,
   wherein the molar ratio of a) to b) in the range of 6:1 to 3:1, and optionally
c) water in the range of ≥ 0.01wt.% to ≤ 5 wt. % to the total weight of the composition,

In another preferred embodiment, the presently claimed invention relates to a composition comprising:
a) L-menthol, and
b) pyruvic acid,
   wherein the molar ratio of a) to b) in the range of 6:1 to 3:1, and
c) water in the range of ≥ 0.01wt.% to ≤ 5 wt. % to the total weight of the composition,
   wherein the composition remains in a liquid state at a temperature in the range of 22°C to°50 C normal atmospheric pressure

### Method

M1-ln an embodiment, the presently claimed invention relates to a method for maintaining L-menthol in a liquid state which comprises at least the step of:
   (I) mixing L-menthol with at least one hydrogen bond donor in a molar ratio of 10:1 to 1:1.
M2-In an embodiment, the presently claimed invention relates to a method for maintaining L-menthol in a liquid state which comprises at least the step of:
   (I) mixing L-menthol with at least one hydrogen bond donor in a molar ratio of 10:1 to 1:1, preferably 8:1 to 2:1, more preferably 6:1 to 3:1.
M3-In an embodiment, the presently claimed invention relates to a method for maintaining L-menthol in a liquid state, which comprises at least the step of:
   (I) mixing L-menthol with lactic acid in a molar ratio of 10:1 to 1:1, preferably 8:1 to 2:1, more preferably 6:1 to 3:1.
M4-In an embodiment, the presently claimed invention relates to a method for maintaining L-menthol in a liquid state, which comprises at least the step of:
   (I) mixing L-menthol with pyruvic acid in a molar ratio of 10:1 to 1:1, preferably 8:1 to 2:1, more preferably 6:1 to 3:1.

In a further embodiment, the presently claimed invention relates to the methods in embodiments M1- M4 wherein mixing L-menthol with at least one hydrogen bond donor or pyruvic acid or lactic acid is optionally mixed with water that the obtained mixture has a water content in the range of ≥ 0.01wt.% to ≤ 5 wt. % of the total weight of the mixture

In an embodiment, the presently claimed invention relates to the methods in embodiments M1-M4 wherein L-menthol is in a liquid state at a temperature in the range of 22°C to°50 C at normal atmospheric pressure for a period of more than 1 year.

In a further embodiment, the presently claimed invention relates to the methods in embodiments M1- M4 wherein L-menthol is in a liquid state at a temperature in the range of 22°C to°50 C at normal atmospheric pressure for a period of more than 6 months

In a further embodiment, the presently claimed invention relates to the methods in embodiments M1- M4 wherein L-menthol is in a liquid state at a temperature in the range of 22°C to°50 C at normal atmospheric pressure for a period of more than 3 months

### Process

The presently claimed invention also relates to a process for preparing the composition.

The process for preparing the composition comprises a) at least one heating step to a temperature ≥ 40 °C to ≤ 100 °C of either the hydrogen bond donor or the L-menthol or both together to obtain a clear liquid, b) a mixing step to achieve a homogeneous mixture and c) a cooling step, wherein optionally, water is added at least in one of the aforementioned steps a) to c) to adjust the water content of the final composition to a range of ≥ 0.01wt.% to ≤ 5 wt. % by total weight of the final composition.

Preferably, the process comprises a) at least one heating step to a temperature in the range of ≥ 40 °C to ≤ 80 °C of either the hydrogen bond donor or the menthol or both together to obtain a clear liquid, b) a mixing step to achieve a homogeneous mixture and c) a cooling step.

In an embodiment of the presently claimed invention, water is added to the composition along with L- menthol and a hydrogen bond donor to adjust the water content of the final composition to a range of ≥ 0.01wt.% to ≤ 5 wt. % by total weight of the final composition.

L-menthol and/or the at least one hydrogen bond donor can contain water. This water content may result from storage and/or preparation of the respective compounds. If L-menthol and/or the at least one hydrogen bond donor contain water, the water content of these components contributes to the overall water content of the composition.

### Use

In an embodiment, the presently claimed invention relates to the use of the composition as described above in flavoring material preparations, cosmetic preparations, preparations used for nutrition, oral hygiene preparations, dental hygiene preparations, perfumes, pharmaceutical preparations, dermatological product preparations, encapsulated menthol-containing preparations, and household product preparations.

In another embodiment, the presently claimed invention relates to a product containing the composition according to the presently claimed invention as described above, wherein the product is selected from the group consisting of flavoring products, cosmetic products, nutritional products, oral hygiene products, dental hygiene products, perfumes, pharmaceutical products, dermatological products, encapsulated menthol-containing products, and household products.

Accordingly, the mixtures according to the presently claimed invention are advantageously particularly suitable as an ingredient of flavoring material preparations or as an ingredient of products that are to be flavored. The mixtures according to the invention are suitable in particular for use in flavoring material preparations, in cosmetic preparations and preparations used for nutrition and/or pleasure, in particular those that conventionally contain (crystalline) menthol.

In an embodiment, the presently claimed invention relates to a product,
(i) comprising a composition according to the presently claimed invention and further ingredients or
(ii) which is prepared by mixing or contacting a composition according to the presently claimed invention with further ingredients,
   wherein the further ingredients of the product do not contain menthol.

In yet another embodiment of the presently claimed invention, the product is selected from the group consisting of flavoring material preparations, cosmetic preparations, preparations used for nutrition, preferably preparations ready for consumption or use, in particular beverages and chewable foodstuffs, in particular chewing gums and chewable sweets (e.g. refreshing sweets), oral hygiene products and dental hygiene products, in particular toothpaste and dental-hygiene chewing gums, perfumes (mixtures of odoriferous substances), pharmaceutical and dermatological products, encapsulated menthol-containing preparations and household products.

In pharmaceutical and dermatological products and cosmetic preparations, a composition according to the presently claimed invention preferably acts as a penetration enhancer. A penetration enhancer is a substance that improves the entry of an active substance into or passage of an active substance through the skin barrier or portions of the skin barrier, in particular the stratum corneum.

In an embodiment of the presently claimed invention, a product according to the presently claimed invention is, according to a first aspect, preferably a flavoring material preparation, which apart from the composition according to the presently claimed invention comprises, as one or a plurality of further ingredients, one or a plurality of further flavoring materials.

A flavoring material preparation according to the presently claimed invention is preferably a flavoring material preparation that is used for flavoring oral hygiene products, dental hygiene products and chewing gums.

In a further embodiment of the presently claimed invention, a product according to the presently claimed invention, as described above, is preferably a cosmetic preparation or a preparation used for nutrition. Said product is preferably an oral hygiene product, a dental hygiene product or a chewing gum.

In yet another embodiment of the presently claimed invention, a product according to the presently claimed invention is preferably a cosmetic preparation. A cosmetic preparation that is preferred according to the presently claimed invention is a dermatological preparation, which (apart from the mixture according to the presently claimed invention that it contains) has the usual composition and preferably serves for cosmetic, in particular dermatological light protection, for the treatment, care and cleaning of the skin and/or of the hair or as make-up product in decorative cosmetics. Correspondingly, such preparations can, depending on their structure, be used for example as skin protective cream, day cream or night cream, eye cream, sunscreen or after-sun lotion, nourishing cream, care mask, gel-pads, face lotion, moist care and cleaning wipes, cleansing milk, cleansing soap, foam or shower gel, deodorants, antiperspirants, hair shampoos, hair care agents, hair conditioners, hair colorings, hair styling agents and preferably as emulsion, lotion, milk, fluid, cream, aqueous-dispersion gel, balm, spray, alcoholic or aqueous-alcoholic solution, foam, powder, liquid soap, soap bar, shampoo, roll-on, stick or make-up. In hair treatment agents, use is preferably directed towards the scalp.

For use, the cosmetic preparations according to the invention are applied in a sufficient amount, in the usual manner for cosmetics, on the skin and/or the hair.

Correspondingly, cosmetic preparations according to the presently claimed invention can, depending on their structure, preferably be used as skin care agents, e.g. skin protective cream, day cream or night cream, sunscreen agents, after-sun preparations (e.g. after-sun lotion), care mask, gel-pads, face lotion, moist care and/or impregnating solution, e.g. for cosmetic wipes, cleaning wipes, cleansing soap, foam or shower gel, liquid soap, soap bar, shampoo (e.g. 2-in-1 shampoo, anti-dandruff shampoo, baby shampoo, shampoo for dry scalp, shampoo concentrate), hair care agents, hair conditioners, hair colourings, hair rinse, hair styling agents (e.g. hair gel), deodorants, antiperspirants (e.g. roll-on or stick), shaving preparations (e.g. aftershave balm, pre-shave or aftershave lotion) or as make-up remover.

A cosmetic preparation according to the presently claimed invention is in the form of an emulsion, lotion, fluid, cream, micro-emulsion, gel (e.g. aqueous or aqueous-dispersion gel), balsam, pump spray, alcoholic or aqueous-alcoholic solution.

Substances and excipients that a product presently claimed according to the invention can (additionally) contain are for example:
Preservatives, preferably those mentioned in US 2006/0089413, abrasives, anti-acne agents and agents for reducing sebum, preferably those mentioned in WO 2008/046791, agents against skin ageing, preferably those mentioned in WO 2005/123101, antibacterial agents, anti-cellulitis agents, antidandruff agents, preferably those mentioned in WO 2008/046795, anti-inflammatory agents, agents preventing irritation, anti-irritants (anti-inflammatory agents, irritation-inhibiting agents and agents preventing irritation), preferably those mentioned in WO 2007/042472 and US 2006/0089413, antimicrobial agents, preferably those mentioned in WO 2005/123101, antioxidants, preferably those mentioned in WO 2005/123101, astringents, antiseptics, antistatic agents, binders, buffers, carriers, preferably those mentioned in WO 2005/123101, chelating agents, preferably those mentioned in WO 2005/123101, cell stimulants, cleansing agents, care agents, depilatory agents, surface active substances, deodorants and antiperspirants, preferably those mentioned in WO 2005/123101, plasticizers, emulsifiers, preferably those mentioned in WO 2005/123101, enzymes, essential oils, preferably those mentioned in US 2008/0070825, insect repellents, preferably those mentioned in WO 2005/123101, fibres, film-forming agents, fixatives, foaming agents, foam stabilizers, antifoaming agents, foam boosters, fungicides, gelling agents and gel-forming agents, preferably those mentioned in WO 2005/123101, hair care agents, hair shaping agents, hair-straightening agents, moisture regulators (hydrating agents, moisturizers and/or humectants), preferably those mentioned in WO 2005/123101, osmolytes, preferably those mentioned in WO 2005/123101, compatible solutes, preferably those mentioned in WO 01/76572 and WO 02/15686, bleaching agents, strengthening agents, stain removers, optical brighteners, impregnating agents, dirt-repelling agents, agents reducing friction, lubricants, moisturizing creams, ointments, opacifiers, plasticizers, covering agents, polishes, gloss agents, polymers, preferably those mentioned in WO 2008/046676, powders, proteins and protein hydrolysates, preferably those mentioned in WO 2005/123101 and WO 2008/046676, refatting agents, abrading agents, skin calmatives, skin cleansing agents, skin care agents, skin healing agents (skin repair agents), preferably containing cholesterol and/or fatty acids and/or ceramides and/or pseudo ceramides, and preferably those mentioned in WO 2006/053912, skin lightening agents, preferably those mentioned in WO 2007/110415, skin protectants, skin softening agents, skin cooling agents, preferably those mentioned in WO 2005/123101, skin warming agents, preferably those mentioned in WO 2005/123101, stabilizers, UV-absorbing agents and UV filters, preferably those mentioned in WO 2005/123101, benzylidene-beta-dicarbonyl compounds, preferably those mentioned in WO 2005/107692, alpha-benzoyl cinnamic acid nitriles, preferably those mentioned in WO 2006/015954, AhR receptor antagonists, preferably those mentioned in WO 2007/128723 and WO 2007/060256, detergents, fabric softeners, suspending agents, skin tanning agents, preferably those mentioned in WO 2006/045760, thickeners, vitamins, preferably those mentioned in WO 2005/123101, oils, waxes and fats, preferably those mentioned in WO 2005/123101, phospholipids, preferably those mentioned in WO 2005/123101, fatty acids (saturated fatty acids, mono- or polyunsaturated fatty acids, α-hydroxy acids, polyhydroxy fatty acids), preferably those mentioned in WO 2005/123101, liquefiers, dyes and colour-protecting agents and pigments, preferably those mentioned in WO 2005/123101, anticorrosive agents, flavourings and flavouring substances and odoriferous substances, preferably those listed in S. Arctander, Perfume and Flavor Chemicals, self-published, Montclair, N.J., 1969 and Surburg, Panten, Common Fragrance and Flavor Materials, 5th edition, Wiley-VCH, Weinheim 2006, in particular those explicitly mentioned in US 2008/0070825, alcohols and polyols, preferably those mentioned in WO 2005/123101, surfactants, preferably those mentioned in WO 2005/123101, animal extracts, yeast extracts, extracts of algae or microalgae, electrolytes, liquefiers, organic solvents, preferably those mentioned in WO 2005/123101, or silicones and silicone derivatives, preferably those mentioned in WO 2008/046676.

In a further embodiment of the presently claimed invention, products according to the presently claimed invention that can be taken orally are also advantageous, e.g. in the form of tablets (e.g. film-coated tablets), sugar-coated tablets, capsules (e.g. gelatine capsules), granules, juices, solutions, emulsions, micro-emulsions, sprays or products consumable orally in other forms or in the form of foodstuffs.

In an embodiment of the presently claimed invention, products as described above, in particular flavouring material preparations, can be in encapsulated form, wherein they are preferably encapsulated with a solid shell material, which is preferably selected from starch, degraded or chemically or physically modified starch (in particular dextrins and maltodextrins), gelatines, gum arabic, agar-agar, ghatti gum, gellan gum, modified and unmodified celluloses, pullulan, curdlan, carrageenans, alginic acid, alginates, pectin, inulin, xanthan gum and mixtures of two or a plurality of the aforementioned substances.

### Advantages

a) The composition according to the presently claimed invention advantageously permits an easily manageable and precise metering in the production of flavouring material preparations, in particular flavour concentrates
b) The composition offers better handling of L-menthol during transportation and storage at ambient temperature.
c) No special equipment for transportation of L-menthol is needed.
d) Another advantage of the composition according to the presently claimed invention is that these only comprises GRAS (Generally Recognised as Safe) excipients which are preferred during its use in the formulation of (finished) products.

### Embodiments

1. A composition comprising:
   a) L-menthol,
   b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
      wherein the molar ratio of a) to b) in the range of 10:1 to 1:1.
2. The composition according to embodiment 1, wherein the composition further comprises water in the range of ≥ 0.01wt.% to ≤ 5 wt. % to the total weight of the composition.
3. The composition according to any of the embodiments 1 or 2, wherein the at least one hydrogen bond donor is selected from the group consisting of C2-C3 aliphatic carboxylic diacids, C2-C4 aliphatic hydroxy carboxylic acids, C3-C4 aliphatic keto carboxylic acids, aromatic carboxylic acids, aromatic carboxylic diacids, phenols and C3-C4 aliphatic diols.
4. The composition according to embodiment 3, wherein the at least one hydrogen bond donor is selected from the group consisting of oxalic acid, glycolic acid, malonic acid, pyruvic acid, D, L- lactic acid, L-lactic acid, benzoic acid, toluic acid, phthalic acid, salicylic acid, propyl gallate, butylated hydroxy anisole, butylated hydroxy toluene, 1,2-butanediol, 1,3-butanediol, 1,4-butanediol, 2,3-butanediol, 1,3-propanediol and 1,2-propanediol.
5. The composition according to embodiment 4, wherein the at least one hydrogen bond donor is selected from the group consisting of pyruvic acid, D, L -lactic acid and L-lactic acid.
6. The composition according to any of the embodiments 1 to 5, wherein the molar ratio a) to b) is in the range of 8:1 to 2: 1
7. The composition according to embodiment 6, wherein the molar ratio a) to b) is in the range of 6:1 to 3: 1
8. The composition according to any of the embodiments 1 to 7, wherein the amount of L-menthol is in the range of ≥ 70 wt. % to ≤ 95 wt. %, based on the total weight of the composition.
9. The composition according to embodiment 8, wherein the amount of L-menthol is in the range of ≥ 80 wt. % to ≤ 90 wt. %, based on the total weight of the composition.
10. The composition according to any of the embodiments 1 to 7, wherein the amount of the at least one hydrogen bond donor is in the range of ≥ 4.5 wt.% to ≤ 25 wt.%, based on the total weight of the composition.
11. The composition according to embodiment 10, wherein the amount of the at least one hydrogen bond donor is in the range of ≥ 9.5 wt. % to ≤ 20 wt. %, based on the total weight of the composition.
12. The composition according to any of the embodiments 1 to 11, wherein the composition remains in a liquid state at a temperature in the range of 22°C to 50°C at normal atmospheric pressure
13. A process for preparing the composition according to any of the embodiments 1 to 12, wherein the process comprises a) at least one heating step to a temperature ≥ 40 °C to ≤ 100 °C of either the at least one hydrogen bond donor or the L-menthol or both together to obtain a clear liquid, b) a mixing step to achieve a homogeneous mixture of the at least one hydrogen bond donor and L-menthol and c) a cooling step, wherein optionally, water is added at least in one of the aforementioned steps to adjust the water content of the final composition to a range of ≥ 0.01wt.% to ≤ 5 wt. % by total weight of the final composition .
14. A method for maintaining L-menthol in a liquid state which comprises at least the step of:
   (I) mixing L-menthol with at least one hydrogen bond donor in a molar ratio of 10:1 to 1:1 and optionally adding water that the so obtained mixture has a water content in the range of ≥ 0.01wt.% to ≤ 5 wt. % of the total weight of the mixture.
   (II) keeping the mixture at a temperature in the range of 22°C to 50°C
   (III) keeping the mixture at atmospheric pressure.
15. The method according to embodiment 14, wherein the at least one hydrogen bond donor has Hansen solubility parameters of δH ≥ 8.0 MPa ½, δD ≥ 16.0 MPa½ and δP ≥ 2.5 MPa½.
16. The method according to embodiment 15, wherein the at least one hydrogen bond donor has Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½.
17. The method according to any of the embodiments 14 to 16, wherein the at least one hydrogen bond donor is selected from the group consisting of C2-C3 aliphatic carboxylic diacids, C2-C4 aliphatic hydroxy carboxylic acids, C3-C4 aliphatic keto carboxylic acids, aromatic carboxylic acids, aromatic carboxylic diacids, phenols and C3-C4 aliphatic diols.
18. The method according to any of the embodiments 14 to 17, wherein the L- menthol remains in a liquid state at a temperature in the range of 22°C to 50°C at normal atmospheric pressure for a period at least one 1 year.
19. Use of the composition according to any of the embodiments 1 to 12 in flavouring material preparations, cosmetic preparations, preparations used for nutrition, oral hygiene preparations, dental hygiene preparations, perfumes, pharmaceutical preparation, dermatological product preparation, encapsulated menthol-containing preparations, and/or household product preparations.
20. A product comprising a composition according to any of the embodiments 1 to 12 and active ingredients, wherein the active ingredients are different from menthol.
21. The product according to embodiment 20, wherein the product is selected from the group consisting of flavouring products, cosmetic products, nutritional products, oral hygiene products, dental hygiene products, perfumes, pharmaceutical products, dermatological products, encapsulated menthol-containing products, and household products.

### Examples

The presently claimed invention is illustrated in detail by non-restrictive working examples which follow. More particularly, the test methods specified hereinafter are part of the general disclosure of the application and are not restricted to the specific working examples.

### Materials

### L-Menthol from BASF SE

### Excipient

- Benzoic acid (contains approx. 0.01% wt./wt. water)
- Butylated hydroxy acetate (BHA) (contains approx. 0.02% wt./wt. water)
- 1,2-propandiol (contains approx. 0.04% wt. /wt. water)
- D-L- lactic acid (85%) (85% w/v solution in water)
- L- lactic acid (85%) (85% w/v solution in water)
- D-lactic acid (90%) (90% w/v solution in water)
- Pyruvic acid (contains approx. 1% wt./ wt. water)

| | **δD** MPa½ | **δP** MPa½ | **δH** MPa½ |
|---|---|---|---|
| Propylene glycol | 16,8 | 9,4 | 23,3 |
| Benzoic acid | 18,2 | 7,0 | 9,8 |
| Lactic acid | 17,0 | 8,3 | 28,4 |
| BHA | 17,3 | 2,8 | 11,2 |
| Pyruvic acid | 16,2 | 11,2 | 8,7 |

### Methods:

DSC; DSC analysis was carried out using a DSC Q2000, TA Instruments the cooling rate was 2K/min and a sample of 8 mg was used. Pressure resistant pans were used.

### Example 1

| Exp. no. | Excipient | L-menthol (%) | Solubility at 60-70 °C | Solubility at RT after 1 hour | Solubility at RT after 24 hours | Solubility at RT after 4 weeks |
|---|---|---|---|---|---|---|
| 1 | Benzoic acid (20%) | 80 | soluble | soluble | soluble | soluble |
| 2 | Butylated hydroxy anisole (20%) | 80 | soluble | soluble | soluble | soluble |
| 3 | 1,2-propandiol (20%) | 80 | soluble | soluble | soluble | soluble |
| 4 | D-L- lactic acid (85%) (20%) | 80 | soluble | soluble | soluble | soluble |
| 5 | L-lactic acid (85%) (20%) | 80 | soluble | soluble | soluble | soluble |
| 6 | Benzoic acid (10%) | 90 | soluble | soluble | soluble | solidified |
| 7 | 1,2-propandiol (10%) | 90 | soluble | soluble | solidified | solidified |
| 8 | D-L- lactic acid (85%) (10%) | 90 | soluble | soluble | soluble | soluble |
| 9 | L-lactic acid (85%) (10%) | 90 | soluble | soluble | soluble | soluble |
| 10 | D-lactic acid (90%) (10%) | 90 | soluble | soluble | soluble | Solidified |

### Process

1) For 10 g batches, the required mass of excipient was weighed in a small glass vessel (with magnetic stir bar and cover). Afterwards it was heated and melted in a drying cabinet at 60-70° C for 30 minutes.
2) At the same time, the L-Menthol was melted in a closed bottle in a water bath to a clear liquid at 50-60°C.
3) The required amount of melted menthol was added to the molten excipient and stirred for several minutes. The formulation was stored in the drying cabinet for 1 h. In case of phase separation, the formulation was stirred again. The formulations were evaluated by means of visual clarity or signs of crystallization, precipitation or solidification at 60-70°C.
4) The samples were removed from the drying cabinet and evaluated after 1 hour/24 hours, and 4 weeks regarding crystallization, precipitation or solidification.

### Example 2

The samples which remained soluble at room temperature for 4 weeks in example 1 were further subjected to
- freeze thaw cycle, refrigerating at 6-8°C and later at room temperature
- standing the formulation at room temperature for 4 weeks with mechanical stress like stirring to induce nuclei for crystallization.

| Exp. no | Excipient | After keeping in fridge at 6-8 °C | Formulation stood for 24 hours at room temperature | Formulation at room temperature for 4 weeks |
|---|---|---|---|---|
| | | Freeze thaw cycle | | |
| | | Step 1 | Step 2 | |
| 1 | 10 % D, L-Lactic acid in L-Menthol (90%) | crystalized after 2 hours | Crystal/ solid | Liquid |
| 2 | 10 % L-Lactic acid in L-Menthol (90%) | crystalized after 2 hours | Crystal/ solid | Liquid |
| 3 | 9% Pyruvic acid in L-Menthol (91%) | crystalized after 2 hours | Crystal/ solid | Liquid |
| 4 | 10% Pyruvic acid in L-Menthol (90%) | crystalized after 2 hours | Crystal/ solid | Liquid |
| 5 | 15% Pyruvic acid in L-Menthol (85%) | crystallized after 4 hours | Liquid | Liquid |
| 6 | 20% Pyruvic acid in L-Menthol (80%) | crystallized after 4 hours | Liquid | Liquid |

### Example 3

DSC analysis was carried out for the samples of example 2 and following are the results of the analysis

| Exp. no | Excipient | Start of Solidification (°C) | Solidification point (°C) |
|---|---|---|---|
| 1 | 10 % D, L-Lactic acid in L-Menthol (90%) | 14.0 | 12.0 |
| 2 | 10 % L-Lactic acid in L-Menthol (90%) | 14.3 | 12.1 |
| 3 | 9% Pyruvic acid in L-Menthol (91%) | 12.8 | 11.0 |
| 4 | 10% Pyruvic acid in L-Menthol (90%) | 10.1 | 8.3 |
| 5 | 15% Pyruvic acid in L-Menthol (85%) | 3.6 | 0.8 |
| 6 | 20% Pyruvic acid in L-Menthol (80%) | -4.0 | -13.6 |
| 7 | 7% Pyruvic acid in L-Menthol (93%) | 21.4 | 17.2 |
| 8 | 12.5 % Pyruvic acid in L-Menthol (87.5%) | 8.5 | 7.3 |
| 9 | 17% Pyruvic acid in L-Menthol (83%) | 3.0 | 0 |

Thus, it can be seen that
- Pyruvic acid, L-, D, L-lactic acid, benzoic acid, propylene glycol and Butylated hydroxyanisol (BHA) were very efficient in depressing the solidification point of L-menthol.
- Formulation containing 90 %pyruvic acid or lactic acid were stable at room temperature as they stayed in liquid state and were clear at room temperature for over 4 weeks. Even upon mechanical stress to initiate crystallization they remained liquid.
- The mole ratio of L-menthol to lactic acid was in the ratio 5:1 and that of L-menthol: pyruvic acid was 5:1 to 1.67:1.
- Storage of formulations at 6-8°C led to solidification/crystallization. The subsequent storage at room temperature resulted again in liquid formulations when using 15 and 20% pyruvic acid.

## Claims

1. A composition comprising:
a) L-menthol,
b) at least one hydrogen bond donor having Hansen solubility parameters in the range of δH ≥ 8.0 to ≤ 30.0 MPa ½, δD ≥ 16.0 to ≤ 21.0 MPa½ and δP ≥ 2.5 to ≤ 14.0 MPa½,
wherein the molar ratio of a) to b) in the range of 10:1 to 1:1.

2. The composition according to claim 1, wherein the composition further comprises water in the range of ≥ 0.01wt.% to ≤ 5 wt. % to the total weight of the composition.

3. The composition according to any of the claims 1 to 2, wherein the at least one hydrogen bond donor is selected from the group consisting of C2-C3 aliphatic carboxylic diacids, C2-C4 aliphatic hydroxy carboxylic acids, C3-C4 aliphatic keto carboxylic acids, aromatic carboxylic acids, aromatic carboxylic diacids, phenols and C3-C4 aliphatic diols.

4. The composition according to claim 3, wherein the at least one hydrogen bond donor is selected from the group consisting of oxalic acid, glycolic acid, malonic acid, pyruvic acid, D,L-lactic acid, L-lactic acid, benzoic acid, toluic acid, phthalic acid, salicylic acid, propyl gallate, butylated hydroxy anisole, butylated hydroxy toluene, 1,2-butanediol, 1,3-butanediol, 1,4-butanediol, 2,3-butanediol, 1,3-propanediol and 1,2-propanediol.

5. The composition according to claim 4, wherein the at least one hydrogen bond donor is selected from the group consisting of pyruvic acid, D, L -lactic acid and L-lactic acid.

6. The composition according to any of the claims 1 to 5, wherein the molar ratio a) to b) is in the range of 8:1 to 2:1.

7. The composition according to claim 6, wherein the molar ratio a) to b) is in the range of 6:1 to 3:1.

8. The composition according to any of the claims 1 to 7, wherein the amount of L-menthol is in the range of ≥ 70 wt. % to ≤ 95 wt. %, based on the total weight of the composition.

9. The composition according to claim 8, wherein the amount of L-menthol is in the range of ≥ 75 wt. % to ≤ 90 wt. %, based on the total weight of the composition.

10. The composition according to any of the claims 1 to 7, wherein the amount of the at least one hydrogen bond donor is in the range of ≥ 4.5 wt.% to ≤ 25 wt.%, based on the total weight of the composition.

11. The composition according to claim 10, wherein the amount of the at least one hydrogen bond donor is in the range of ≥ 9.5 wt. % to ≤ 20 wt. %, based on the total weight of the composition.

12. The composition according to any of the claims 1 to 11, wherein the composition remains in a liquid state at a temperature in the range of 22°C to 50°C at normal atmospheric pressure.

13. A process for preparing the composition according to any of the claims 1 to 12, wherein the process comprises a) at least one heating step to a temperature ≥ 40 °C to ≤ 100 °C of either the at least one hydrogen bond donor or the L-menthol or both together to obtain a clear liquid, b) a mixing step to achieve a homogeneous mixture of the at least one hydrogen bond donor and L-menthol and c) a cooling step, wherein optionally, water is added at least in one of the aforementioned steps to adjust the water content of the final composition to a range of ≥ 0.01wt.% to ≤ 5 wt. % by total weight of the final composition.

14. A method for maintaining L-menthol in a liquid state which comprises at least the step of:
(I) mixing L-menthol with at least one hydrogen bond donor in a molar ratio of 10:1 to 1:1 and optionally adding water that the so obtained mixture has a water content in the range of ≥ 0.01wt.% to ≤ 5 wt. % of the total weight of the mixture.
(II) keeping the mixture at a temperature in the range of 22°C to 50°C
(III) keeping the mixture at atmospheric pressure.

15. The method according to claim 14, wherein the at least one hydrogen bond donor has Hansen solubility parameters of δH ≥ 8.0 MPa ½, δD ≥ 16.0 MPa½ and δP ≥ 2.5 MPa½.

16. The method according to claim 15, wherein the at least one hydrogen bond donor has Hansen solubility parameters in the range of δH ≥ 8.0 to ≤ 30.0 MPa ½, 5D ≥ 16.0 to ≤ 21.0 MPa½ and δP ≥ 2.5 to ≤ 14.0 MPa½.

17. The method according to any of the claims 14 to 16, wherein the at least one hydrogen bond donor is selected from the group consisting of C2-C3 aliphatic carboxylic diacids, C2-C4 aliphatic hydroxy carboxylic acids, C3-C4 aliphatic keto carboxylic acids, aromatic carboxylic acids, aromatic carboxylic diacids, phenols and C3-C4 aliphatic diols.

18. The method according to any of the claims 14 to 17, wherein the L- menthol remains in a liquid state at a temperature in the range of 22°C to 50°C at normal atmospheric pressure for a period of at least 1 year.

19. Use of the composition according to any of the claims 1 to 12 in flavouring material preparations, cosmetic preparations, preparations used for nutrition, oral hygiene preparations, dental hygiene preparations, perfumes, pharmaceutical preparation, dermatological product preparation, encapsulated menthol-containing preparations, and/or household product preparations.

20. A product comprising a composition according to any of the claims 1 to 12 and active ingredients, wherein the active ingredients are different from menthol.

21. The product according to claim 20, wherein the product is selected from the group consisting of flavouring products, cosmetic products, nutritional products, oral hygiene products, dental hygiene products, perfumes, pharmaceutical products, dermatological products, encapsulated menthol-containing products, and household products.
